(19) Europäisches Patentamt
European Patent Office
Office européen des brevets

(11) Veröffentlichungsnummer : **0 147 742**
**B1**

(12) # EUROPÄISCHE PATENTSCHRIFT

(45) Veröffentlichungstag der Patentschrift : **08.07.87**

(51) Int. Cl.$^4$ : **C 01 B 21/14**

(21) Anmeldenummer : **84115403.2**

(22) Anmeldetag : **14.12.84**

(54) **Stabilisierte Lösungen von Hydroxylamin oder dessen Salzen in Wasser oder Alkoholen sowie deren Herstellung.**

(30) Priorität : **28.12.83 DE 3347260**

(43) Veröffentlichungstag der Anmeldung :
**10.07.85 Patentblatt 85/28**

(45) Bekanntmachung des Hinweises auf die Patenterteilung : **08.07.87 Patentblatt 87/28**

(84) Benannte Vertragsstaaten :
**BE CH DE FR GB IT LI NL**

(56) Entgegenhaltungen :
**US-A- 3 145 082**
**US-A- 3 480 391**
**US-A- 3 480 392**
**US-A- 3 544 270**
**PATENT ABSTRACTS OF JAPAN, Band 7, Nr. 161 (C-176)[1306], 15. Juli 1983**
**PATENT ABSTRACTS OF JAPAN, Band 7, Nr. 161, (C-176)[1306], 15. Juli 1983**
**PATENT ABSTRACTS OF JAPAN, Band 7, Nr. 161 (C179)[1306], 15. Juli 1983**
**PATENT ABSTRACTS OF JAPAN, Band 7, Nr. 161 (C-176)[1306]**

(73) Patentinhaber : **BASF Aktiengesellschaft**
**Carl-Bosch-Strasse 38**
**D-6700 Ludwigshafen (DE)**

(72) Erfinder : **Grosskinsky, Otto-Alfred, Dr. Chem.**
**Semmelweisstrasse 8**
**D-6700 Ludwigshafen (DE)**
Erfinder : **Frommer, Elmar, Dr. Chem.**
**Lisztstrasse 117**
**D-6700 Ludwigshafen (DE)**
Erfinder : **Ritz, Josef, Dr. Chem.**
**Osloer Weg 8**
**D-6700 Ludwigshafen (DE)**
Erfinder : **Thomas, Erwin**
**Borngasse 12**
**D-6713 Freinsheim (DE)**
Erfinder : **Weiss, Franz-Josef, Dr. Chem.**
**Schilfweg 1**
**D-6708 Neuhofen (DE)**

**EP 0 147 742 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Lösungen von Hydroxylammoniumsalzen zersetzen sich langsam bei Raumtemperatur und schneller bei erhöhter Temperatur. Dies gilt in vermehrtem Maße für Lösungen von freiem Hydroxylamin. Es hat nicht an Versuchen gefehlt, Lösungen von Hydroxylamin und dessen Salzen zu stabilisieren, um eine Verbesserung der Lagerfähigkeit zu erreichen. So verwendet man als Stabilisierungsmittel Harnstoffderivate, entsprechend der US-A-3 544 270. Auch Amidoxime wurden bereits für die Stabilisierung von Hydroxylaminlösungen, entsprechend der US-A-3 480 391 beschrieben. Ferner eignen sich Hydroxamsäuren für die Stabilisierung von Hydroxylamin in Lösungen, wie aus der US-A-3 480 392 bekannt ist. Aus der US-A-3 145 082 ist auch schon bekannt, chelatbildende Mittel, wie das Natriumsalz der Ethylendiamintetraessigsäure als Stabilisierungsmittel zu verwenden. Die bislang verwendeten Stabilisierungsmittel sind noch verbesserungsbedürftig.

Es war die technische Aufgabe gestellt, stabilisierte Lösungen von Hydroxylamin oder dessen Salzen zur Verfügung zu stellen, die über einen längeren Zeitraum stabil sind und bei denen insbesondere die Zersetzung von freiem Hydroxylamin minimiert wird.

Diese Aufgabe wird gelöst durch stabilisierte Lösungen von Hydroxylamin oder dessen Salzen in Wasser oder Alkoholen, gekennzeichnet durch einen Gehalt an Anthocyanen der Formel

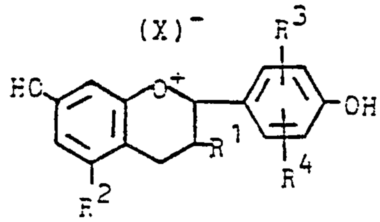

(I)

in der $R^1$ bis $R^4$ jeweils ein Wasserstoffatom, eine Hydroxylgruppe oder eine $C_1$- bis $C_4$-Alkoxygruppe bezeichnen und X für ein Anion einer starken Mineralsäure steht.

Ferner ist ein Gegenstand der Erfindung ein Verfahren zur Herstellung von stabilisierten Lösungen von Hydroxylamin oder dessen Salzen durch Zugabe von Stabilisatoren, dadurch gekennzeichnet, daß man aus der zu stabilisierenden Lösung den darin gelöst enthaltenen molekularen Sauerstoff durch Behandeln mit Stickstoff, der frei von molekularen Sauerstoff ist, entfernt und dann Anthocyane der Formel I zusetzt.

Die gemäß der Erfindung stabilisierten Lösungen von Hydroxylamin oder dessen Salzen haben den Vorteil, daß sie über längere Zeit als bisher stabil sind und insbesondere die Zersetzung von freiem Hydroxylamin auf ein Minimum reduziert wird.

Erfindungsgemäß geht man von Lösungen von Hydroxylamin oder dessen Salzen in Wasser oder Alkoholen, z. B. $C_1$- bis $C_4$-Alkanolen aus. Geeignete Salze des Hydroxylamins sind beispielsweise solche mit starken Mineralsäuren, wie Schwefelsäure, Salpetersäure, Chlorwasserstoffsäure oder solche mit Fettsäuren, z. B. Essigsäure oder Propionsäure. Aufgrund der verschiedenen Löslichkeiten liegt Hydroxylamin vorzugsweise gelöst in Wasser oder Alkoholen vor, während dessen Salze vorzugsweise als wäßrige Lösungen vorliegen. Der Gehalt an Hydroxylamin oder dessen Salze beträgt in der Regel von 10 bis 70 Gew.- %. Die verwendeten Lösungen von Hydroxylamin haben in der Regel einen pH-Wert von 8 bis 11. Besonders bevorzugt geht man von Lösungen von Hydroxylamin in Wasser aus.

Als Stabilisatoren verwendet man Anthocyane der Formel

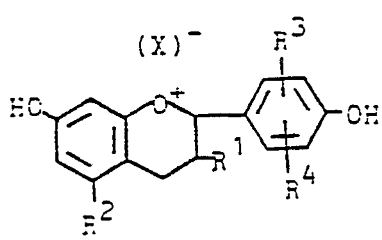

(I)

in der $R^1$ bis $R^4$ jeweils ein Wasserstoffatom, eine Hydroxylgruppe oder eine $C_1$- bis $C_4$-Alkoxygruppe bezeichnen und X für ein Anion einer starken Mineralsäure steht. Vorzugsweise steht X für ein Chlorid- oder Sulfatanion. In besonders bevorzugten Anthocyanen der Formel I bezeichnen $R^1$ und $R^4$ jeweils ein Wasserstoffatom, eine Hydroxylgruppe oder eine Methoxygruppe und X steht für ein Chloridanion. Die Anthocyane können auch als Glykoside vorliegen. Geeignete Anthocyane werden beispielsweise beschrieben in Angewandte Chemie 68 (1956), Seiten 110 und 111. Geeignete Verbindungen Apigenin, Pelargonidin, Cyanidin, Delphinidin, Päonidin, Petunidin, Malvidin.

Vorteilhaft wendet man die Anthocyane der Formel I in Mengen von 0,005 bis 1 Gew.- %,

insbesondere 0,01 bis 0,1 Gew.-%, bezogen auf die zu stabilisierende Lösung an. Es hat sich ferner bewährt, wenn man zusätztzlich Polyhydroxybenzole, insbesondere Pyrogallol mitverwendet. Polyhydroxybenzole werden vorteilhaft in Mengen von 0,005 bis 0,1 Gew.-%, bezogen auf die zu stabilisierende Lösung zugesetzt. Es ist bemerkenswert, daß durch die kombinierte Anwendung von Anthocyanen der Formel I und Polyhydroxybenzolen eine synergistische Wirkung erzielt wird.

Stabilisierte Lösungen von Hydroxylamin oder dessen Salzen in Wasser oder Alkoholen werden erfindungsgemäß hergestellt, indem man aus den zu stabilisierenden Lösungen zunächst den darin gelöst enthaltenen molekularen Sauerstoff durch Behandeln mit Stickstoff, der frei von molekularem Sauerstoff ist, verdrängt. Dies erzielt man beispielsweise dadurch, daß man durch die zu stabilisierende Lösung sauerstoffreien Stickstoff leitet, z. B. für 5 bis 10 Minuten. Der hierfür verwendete Stickstoff hat vorteilhaft einen Gehalt an molekularem Sauerstoff von weniger als 2 ppm. Dann gibt man Anthocyane der Formel I und gegebenenfalls Hydroxybenzole zu und löst diese in der zu stabilisierenden Lösung auf. Vorteilhaft hält man hierbei eine Temperatur von 5 bis 40 °C ein. Es ist auch möglich, die Stabilisatoren bereits in gelöstem Zustand den zu stabilisierenden Lösungen zuzufügen.

Es versteht sich, daß es von Vorteil ist, eine Kontamination der zu stabilisierenden Lösungen mit Schwermetallen, insbesondere Kupfer oder Edelmetallen zu vermeiden, da diese die Zersetzung von Hydroxylamin katalysieren. Ferner ist es von Vorteil, energiereiche Strahlung durch geeignet eingefärbte Glasbehälter auszuschalten. Schließlich ist es vorteilhaft, die stabilisierten Lösungen bei Temperaturen von $< 40$ °C, z. B. bei Temperaturen von 5 bis 20 °C aufzubewahren.

Stabilisierte Lösungen von Hydroxylamin oder dessen Salzen eignen sich zur Herstellung von Oximen.

Der erfindungsgemäße Gegenstand sei an folgenden Beispielen veranschaulicht.

### Beispiel

Eine wäßrige Lösung von Hydroxylamin wird bei 20 °C 10 Minuten mit sauerstoffreiem Stickstoff gespült und dann der Stabilisator zugefügt. Die Konzentration an Hydroxylamin, die zugegebene Menge und Art des Stabilisators sowie die in Abhängigkeit von Zeit und Temperatur erzielten Ergebnisse sind aus folgender Tabelle zu entnehmen :

Tabelle

| Stabilisator | °C | | | | |
|---|---|---|---|---|---|
| 50 ppm Cyanidin | 5 | 0 | 743 | 1198 | Stunden |
| | | 110,88 | 110,73 | 110,50 | g/l $NH_2OH$ |
| 50 ppm Cyanidin | 20 | 0 | 404 | 1195 | Stunden |
| | | 110,25 | 109,65 | 109,39 | g/l $NH_2OH$ |
| 50 ppm Cyanidin | 40 | 0 | 359 | 1194 | Stunden |
| | | 110,25 | 109,70 | 108,67 | g/l $NH_2OH$ |

## Patentansprüche

1. Stabilisierte Lösungen von Hydroxylamin oder dessen Salzen in Wasser oder Alkoholen gekennzeichnet durch einen Gehalt an Anthocyanen der Formel

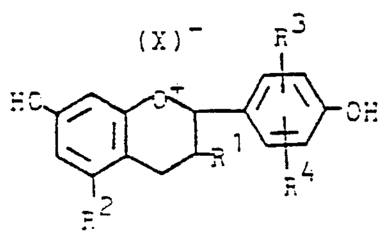

(I)

in der $R^1$ bis $R^4$ jeweils ein Wasserstoffatom, eine Hydroxylgruppe oder eine $C_1$- bis $C_4$-Alkoxygruppe bezeichnen und X für ein Anion einer starken Mineralsäure steht.

2. Stabilisierte Lösungen nach Anspruch 1, gekennzeichnet durch einen Gehalt an Anthocyanen der Formel I, in der $R^1$ bis $R^4$ jeweils ein Wasserstoffatom, eine Hydroxylgruppe oder eine Methoxygruppe bezeichnen und X für ein Chloridanion steht.

3. Stabilisierte Lösungen nach den Ansprüchen 1 bis 2, gekennzeichnet durch einen Gehalt von 0,005 bis 1 Gew.-% an Anthocyanen der Formel I, bezogen auf die Menge der zu stabilisierenden Lösung.

4. Stabilisierte Lösungen nach den Ansprüchen 1 bis 3, gekennzeichnet durch einen zusätzlichen Gehalt an Pyrogallol.

5. Verfahren zur Herstellung von stabilisierten Lösungen von Hydroxylamin oder dessen Salze in Wasser oder Alkoholen durch Zusatz von Stabilisatoren, dadurch gekennzeichnet, daß man aus den zu stabilisierenden Lösungen den darin gelöst enthaltenen molekularen Sauerstoff durch Behandeln mit Stickstoff, der frei von molekularem Sauerstoff ist, entfernt und dann Anthocyane der Formel I zusetzt.

**Claims**

1. A stabilized solution of hydroxylamine or its salts in water or an alcohol, which contains an anthocyan of the formula

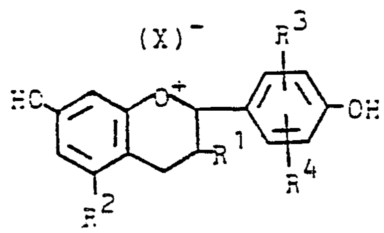

(I)

where $R^1$, $R^2$, $R^3$ and $R^4$ are each hydrogen, hydroxyl or $C_1$-$C_4$-alkoxy, and X is an anion of a strong mineral acid.

2. A stabilized solution as claimed in claim 1, which contains an anthocyan of the formula I, where $R^1$, $R^2$, $R^3$ and $R^4$ are each hydrogen, hydroxyl or methoxy and X is a chloride anion.

3. A stabilized solution as claimed in claims 1 and 2, which contains from 0.005 to 1 % by weight, based on the amount of the solution to be stabilized, of an anthocyan of the formula I.

4. A stabilized solution as claimed in claims 1 to 3, which additionally contains pyrogallol.

5. A process for the preparation of a stabilized solution of hydroxylamine or its salts in water or an alcohol by the addition of a stabilizer, wherein the molecular oxygen dissolved in the solution to be stabilized is removed from this solution by treatment with nitrogen which is free of molecular oxygen, and an anthocyan of the formula I is then added.

**Revendications**

1. Solutions stabilisées d'hydroxylamine ou ses sels dans l'eau ou les alcools caractérisées par une teneur en anthocyanes de formule

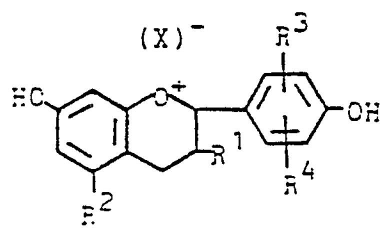

(I)

dans laquelle $R^1$ à $R^4$ représentent chacun un atome d'hydrogène, un groupe hydroxyle ou un groupe alcoxy en $C_1$ à $C_4$ et X est mis pour un anion ou un acide minéral fort.

2. Solutions stabilisées selon la revendication 1, caractérisées par une teneur en anthocyanes de formule I, dans laquelle $R^1$ à $R^4$ représentent chacun un atome d'hydrogène, un groupe hydroxyle ou un groupe méthoxy et X est mis pour un anion chlorure.

3. Solutions stabilisées selon les revendications 1 à 2, caractérisées par une teneur de 0,005 à 1 % en poids en anthocyanes de formule I, rapportée à la quantité de solution à stabiliser.

4. Solutions stabilisées selon les revendications 1 à 3, caractérisées par une teneur additionnelle en pyrogallol.

5. Procédé de préparation de solutions stabilisées d'hydroxylamine ou ses sels dans l'eau ou les alcools par addition de stabilisants, caractérisé en ce que l'on élimine des solutions à stabiliser l'oxygène moléculaire qui y est contenu dissous, par traitement avec de l'azote qui est exempt d'oxygène moléculaire, puis on ajoute de l'anthocyane de formule I.